(19) **Europäisches Patentamt**

**European Patent Office**

**Office européen des brevets**

(11) **EP 0 892 908 B1**

(12) **EUROPEAN PATENT SPECIFICATION**

(45) Date of publication and mention
of the grant of the patent:
**14.05.2003 Bulletin 2003/20**

(51) Int Cl.7: **G01B 7/14**, G01B 7/004

(21) Application number: **97908474.6**

(86) International application number:
**PCT/IL97/00100**

(22) Date of filing: **18.03.1997**

(87) International publication number:
**WO 97/036143 (02.10.1997 Gazette 1997/42)**

(54) **MUTUAL INDUCTION CORRECTION**

GEGENINDUKTIONSKORREKTUR

CORRECTION D'INDUCTIONS MUTUELLES

(84) Designated Contracting States:
**DE ES FR GB IT NL**

(30) Priority: **26.03.1996 US 14084 P**

(43) Date of publication of application:
**27.01.1999 Bulletin 1999/04**

(73) Proprietor: **Biosense, Inc.**
**Miami, Florida 33173 (US)**

(72) Inventors:
• **GOVARI, Assaf**
  **26272 Kiriat Haim (IL)**

• **OSADCHY, Daniel**
  **34731 Haifa (IL)**
• **FENSTER, Maier**
  **49600 Petach Tikva (IL)**

(74) Representative: **Mercer, Christopher Paul**
**Carpmaels & Ransford**
**43, Bloomsbury Square**
**London WC1A 2RA (GB)**

(56) References cited:
**EP-A- 0 339 723**     **US-A- 5 068 608**
**US-A- 5 453 687**

**Description**

FIELD OF THE INVENTION

The present invention relates generally to apparatus for generating and detecting electromagnetic fields, and specifically to non-contact, electromagnetic methods and devices for tracking the position and orientation of an object.

BACKGROUND OF THE INVENTION

Non-contact electromagnetic tracking systems are well known in the art, with a wide range of applications.

For example, U.S. patent 4,054,881 describes a tracking system using three coils to generate electromagnetic fields in the vicinity of the object. The fields generated by these three coils are distinguished from one another by open loop multiplexing of time, frequency or phase. The signal currents flowing in three orthogonal sensor coils are used to determine the object's position, based on an iterative method of computation.

U.S. patent 5,391,199, filed July 20, 1993, which is assigned to the assignee of the present application, describes a system for generating three-dimensional location information regarding a medical probe or catheter. A sensor coil is placed in the catheter and generates signals in response to externally applied magnetic fields. The magnetic fields are generated by three radiator coils, fixed to an external reference frame in known, mutually spaced locations. The amplitudes of the signals generated in response to each of the radiator coil fields are detected and used to compute the location of the sensor coil. Each radiator coil is preferably driven by driver circuitry to generate a field at a known frequency, distinct from that of other radiator coils, so that the signals generated by the sensor coil may be separated by frequency into components corresponding to the different radiator coils.

PCT patent application number WO 96/05768, which is assigned to the assignee of the present application, describes a system that generates six-dimensional position and orientation information regarding the tip of a catheter. This system uses a plurality of non-concentric sensor coils adjacent to a locatable site in the catheter, for example near its distal end and a plurality of radiator coils fixed in an external reference frame. These coils generate signals in response to magnetic fields generated by the radiator coils, which signals allow for the computation of six location and orientation coordinates. As in the case of the '539 patent application described above, the radiator coils operate simultaneously at different frequencies, for example at 1000, 2000 and 3000 Hz, respectively.

European patent application EP-A-0339723 describes a magnetic resonance apparatus having a magnet system for generating a steady magnetic field and an rf coil system for generating gradient fields. In the rf coil system, each coil can be detuned for a given frequency of another coil. Therefore, mutual influencing is avoided.

The above tracking systems rely on separation of position-responsive signals into frequency components, wherein each such component is assumed to correspond uniquely to a single radiator coil, in a known position, radiating in a narrow, well-defined frequency band. In practice, however, the radiator coils also generate magnetic fields at the frequencies outside the desired bands, for example due to mutual inductance effects. These mutually-induced fields lead to errors in determining the position of the object being tracked.

SUMMARY OF THE INVENTION

It is an object of the present invention to provide improved electromagnetic radiator coils and driver circuitry therefor, for use in conjunction with object tracking systems, so as to increase the accuracy of object tracking.

It is a further object of the present invention to provide magnetic field generator coils and associated driver circuitry that generate fields of having narrow bandwidths in the frequency domain.

In the present invention, narrowed field bandwidth is achieved by eliminating mutual inductance effects among a plurality of coils, which generate magnetic fields at different frequencies.

The present invention provides apparatus for tracking an object by generating magnetic fields, comprising a plurality of radiator coils; driver circuitry coupled thereto, which drives the coils so as to generate magnetic fields at a plurality of driving frequencies; wherein each of the plurality of radiator coils generates a field substantially only at a single, respective driving frequency; characterised by: circuitry associated with at least one of said plurality of radiator coils, wherein said circuitry generates magnetic fields for substantially eliminating magnetic fields generated by the at least one coil in response to fields generated by the other coils.

In some preferred embodiments of the present invention, one or more additional shimming coils are adjacent to each of the radiator coils. Preferably the shimming coils are interwound with windings of the radiator coils. The shimming coils of each radiator coil are driven by driver circuitry so as to generate magnetic fields that are substantially equal in amplitude and frequency, and opposite in direction, to magnetic field components induced along the axis of that radiator coil by other radiator coils.

In other preferred embodiments of the present invention, driver circuitry is associated with each of the coils and generates electrical driver currents therein, wherein for each coil the current comprises a major component at the coil's respective driving frequency, and minor components at other frequencies. The minor components are substantially equal in amplitude and frequency and 180° out of phase with currents induced in the coil due to magnetic fields generated by other radiator coils, so as to substantially cancel the effect of the induced currents.

In one such preferred embodiment of the present invention, driver circuitry includes sensing apparatus, which measures the amplitude, frequency and phase of induced currents in a coil. The driver circuitry further includes an adaptive current supply, which receives the amplitude, frequency and phase data measured by the sensing apparatus, and generates the out of-phase minor current components to substantially cancel the effect of the induced currents.

In still other preferred embodiments of the present invention, driver circuitry comprises ideal current sources, which function to maintain a constant current in each of the coils at its fixed, respective driving frequency.

The circuitry for eliminating magnetic fields preferably comprises one or more shimming coils, which are preferably interwound with the at least one of said plurality of radiator coils. Furthermore, the driver circuitry preferably drives one of the one or more shimming coils associated with the at least one of said plurality of radiator coils at the respective driving frequency of another one of said plurality of radiator coils.

Preferably the circuitry for eliminating includes an adaptive current supply, which generates electrical current in the at least one of the plurality of radiator coils at the respective driving frequency of another one of the plurality of radiator coils.

Preferably the circuitry for eliminating further includes a current analyzer, coupled to the adaptive current supply, which current analyzer measures parasitic electrical current flowing in the at least one of the plurality of radiator coils at the respective driving frequency of another one of the plurality of radiator coils, and causes the adaptive current supply to adjust the generated current so as to minimize the parasitic electrical current.

Preferably the driver circuitry is adapted to maintain a constant current in each of the plurality of radiator coils at the single, respective driving frequency thereof.

**BRIEF DESCRIPTION OF THE DRAWINGS**

The present invention will be more fully understood from the following detailed description of the preferred embodiments thereof, taken together with the drawings in which:

Fig. 1 is a schematic representation of an object tracking system operative in accordance with a preferred embodiment of the present invention;

Fig. 2 is a schematic representation of a radiator coil in accordance with a preferred embodiment of the present invention;

Fig. 3 is a schematic representation of coil driver circuitry in accordance with another preferred embodiment of the present invention; and

Fig. 4 is a flow chart illustrating schematically a method of object tracking in accordance with a preferred embodiment of the present invention.

**DETAILED DESCRIPTION OF PREFERRED EMBODIMENTS**

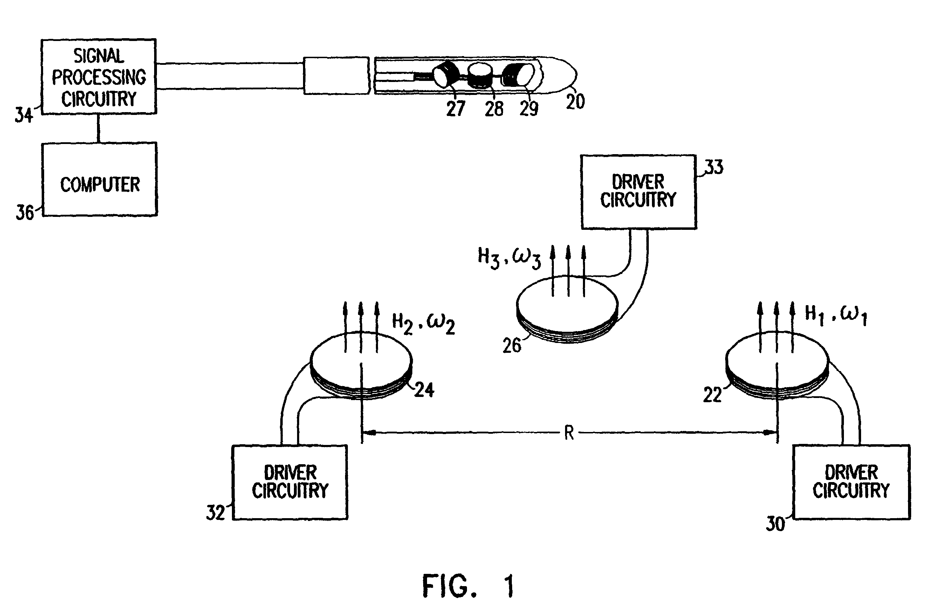

Reference is now made to Fig. 1, which illustrates schematically a system for tracking a probe 20, such as a catheter for medical use, operative in accordance with preferred embodiment of the present invention. As described in U.S. patent 5,391,199, to Ben-Haim, and in PCT patent application number WO 96/05768, which are assigned to the assignee of the present application, the system comprises a plurality of radiator coils 22, 24 and 26. These coils generate respective magnetic fields $\mathbf{H_1}$, $\mathbf{H_2}$ and $\mathbf{H_3}$, at respective frequencies $\omega_1$, $\omega_2$ and $\omega_3$, in the vicinity of probe 20. The probe further includes sensor coils 27, 28 and 29, which generate electrical current signals in response to the magnetic fields, wherein the signals comprise. components at frequencies $\omega_1$, $\omega_2$ and $\omega_3$, whose respective amplitudes are dependent on the position and orientation of probe 20.

The system further comprises driver circuitry 30, 32 and 33, coupled to each of the radiator coils, which drives coils 22, 24 and 26 at respective driving frequencies $\omega_1$, $\omega_2$ and $\omega_3$. The signals generated by sensor coils 27, 28 and 29 are preferably processed by signal processing circuitry 34 and then used by computer 36 to calculate position and orientation coordinates of probe 20.

For the sake of clarity, Fig. 1 shows three radiator coils 22, 24 and 26 and three sensor coils 27, 28 and 29 in a probe 20. It will be understood, however, that the present invention is equally applicable to tracking systems comprising two, four or more radiator coils and one, two or more sensor coils. The present invention may be used in tracking other types of objects, as well.

In the absence of mutual inductance effects, the signals generated by sensor coils 27, 28 and 29 at frequency $\omega_1$ are proportional to the amplitude of the time derivative of the projection of magnetic flux due to field $\mathbf{H_1}$ at probe 20 along each of the respective axes of the sensor coils. The signals generated at frequencies $\omega_2$ and $\omega_3$ are similarly proportional to the projections of $\mathbf{H_2}$ and $\mathbf{H_3}$. Since the direction and amplitude of the magnetic field due to a single such radiator coil can be calculated easily using methods known in the art, the sensor coil signals due to a single radiator coil may be directly related to the sensor coil's distance from and orientation relative to the radiator coil.

In practice, however, magnetic field $\mathbf{H_1}$ generated by radiator coil 22 is not limited in space to an immediate vicinity of probe 20, but also has a non-zero amplitude in a vicinity of coils 24 and 26. As is well known in the art (see, for example, Lorrain and Corson, Electromagnetic Fields and Waves, Second Edition, 1970, pages 343-345), the magnetic flux $\Phi_2$ through coil 24 due to current $I_1$ flowing in coil 22 may be expressed as:

$$\Phi_2 = M_{12}I_1 \tag{1}$$

wherein $M_{12}$ is the mutual inductance of coils 22 and 24, and the voltage induced in coil 24 will be:

$$V_{12} = -M_{12}\frac{dI_1}{dt} \tag{2}$$

Assuming coil 24 to have inductance L and zero resistance, the overall voltage in the coil will be:

$$V_2 = -L\frac{dI_2}{dt} \tag{3}$$

Since driver circuitry 30 and 32 preferably drives coils 22 and 24 with sinusoidal electrical currents at the coils' respective frequencies $\omega_1$ and $\omega_2$, an additional current $I_{12}$ at frequency $\omega_1$ will flow in coil 24, having amplitude given by:

$$I_{12} = \frac{M_{12}\omega_1}{L\omega_2}I_1 \tag{4}$$

Current $I_{12}$ flowing in radiator coil 24 will cause a parasitic magnetic field $\mathbf{H_{12}}$ to be generated at frequency $\omega_1$, whose amplitude will be approximately proportional to the ratio $M_{12}/L$ when $\omega_1 \approx \omega_2$. $M_{12}$ depends on geometrical factors and on the number of turns in each of the coils. It can be shown (see, for example, J.D. Jackson, Classical Electrodynamics, Second Edition, page 263) that for two coplanar current loops of radius a, mutually spaced by a distance R:

$$M_{12} = \mu\pi a\left[\left(\frac{a}{R}\right)^3 + \frac{9}{4}\left(\frac{a}{R}\right)^5 + \frac{375}{64}\left(\frac{a}{R}\right)^7 + \ldots\right] \tag{5}$$

For coils 22 and 24 comprising $n_1$ and $n_2$ turns per unit length, respectively, and having substantially identical lengths $\ell$, inner radii $r_1$ and outer radii $r_2$, and assuming that the magnetic field $\mathbf{H_1}$ is substantially uniform over all the turns of coil 24, we may integrate equation (5) to find the mutual inductance of the coils:

$$M_{12} = \mu\pi n_1 n_2 \ell^2 R^2\left[\frac{1}{5}\left(\frac{r_2^5 - r_1^5}{R^5}\right) + \frac{9}{28}\left(\frac{r_2^7 - r_1^7}{R^7}\right) + \ldots\right] \tag{6}$$

Thus, for example, in a tracking system such as those described in U.S. patent 5,391,199 and in PCT patent application number WO 96/05768, wherein coils 22 and 24 have inner radii of 25 mm and outer radii of approximately 60 mm, and are mutually spaced by 39 cm, it will be observed that $I_{12} = 0.0025(\omega_1/\omega_2)I_1$, which could lead to an error of 0.5% when $\omega_1=2\omega_2$. Thus, the signals generated by sensor coils 27, 28 and 29 at frequency $\omega_1$ will include both a position signal component due to $\mathbf{H_1}$ and a parasitic signal component due to $\mathbf{H_{12}}$, which may introduce errors of up to 1% in determination of the position of probe 20. Additional parasitic signal components at frequency $\omega_1$ will be introduced by mutual inductance in coil 26 and any other radiator coils. Similarly, sensor coil signals at frequencies $\omega_2$ and $\omega_3$ will also include parasitic components.

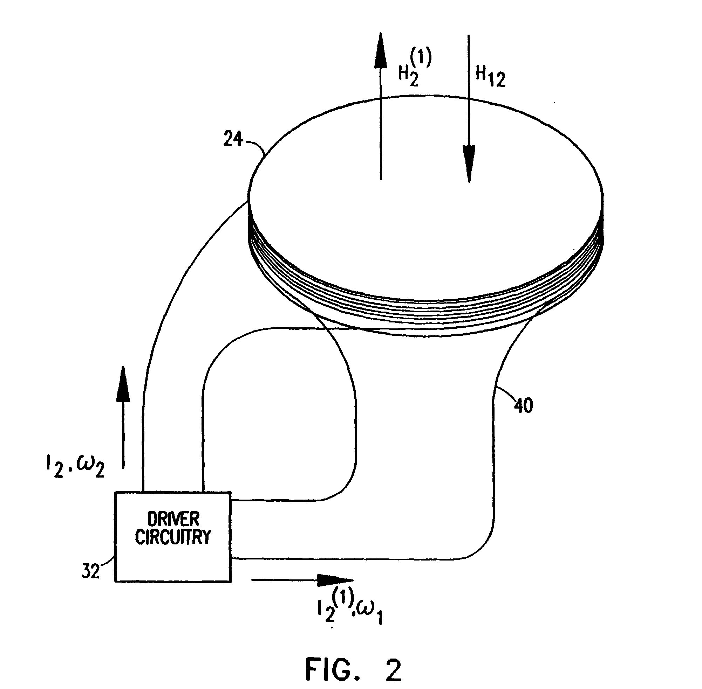

For this reason, in a preferred embodiment of the present invention shown in Fig. 2, one or more shimming coils 40 are adjacent to radiator coil 24, and preferably interwound therewith. Driver circuitry 32 drives coil 24 with current $I_2$ at the coil's driving frequency $\omega_2$. Circuitry 32 further drives shimming coil 40 with current $I_2(1)$ at frequency $\omega_1$, wherein this current causes a magnetic field $\mathbf{H_2^{(1)}}$ to be generated along the axis of coil 24 which is equal in amplitude and 180° out of phase with magnetic field $\mathbf{H_{12}}$. Consequently, mutually-induced current $I_{12}$ will be substantially canceled out by an equal and opposite current induced due to field $\mathbf{H_2^{(1)}}$.

Although, for the sake of clarity, Fig. 2 and the foregoing explanation relate only to a single shimming coil, driven at a single frequency, it will be understood that in preferred embodiments of the present invention, each of coils 22, 24 and 26 will be interwound with at least one shimming coil, driven with appropriate currents and frequencies so as to substantially cancel out mutually-induced currents due to all other coils.

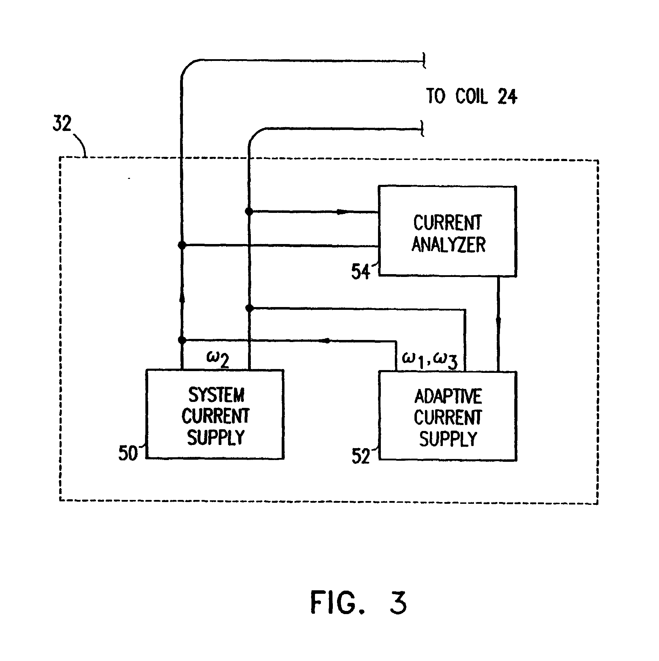

Fig. 3 illustrates schematically an alternative preferred embodiment of the present invention, wherein driver circuitry 32 is adapted to drive coil 24 so as to substantially cancel mutually induced currents. Circuitry 32 comprises system current supply 50, which drives coil 24 at its driving frequency $\omega_2$, and adaptive current supply 52, which generates current to drive coil 24 at the respective frequencies of coils 22 and 26, $\omega_1$ and $\omega_3$, with amplitudes and phases determined by current analyzer 54. The current analyzer samples the current in coil 24, using methods known in the art, and separates the sampled current into frequency components. Analyzer 54 causes adaptive supply 52 to adjust the amplitudes and phases of the generated $\omega_1$ and $\omega_3$ currents so as to minimize the components sensed at frequencies $\omega_1$ and $\omega_3$ in the sampled current from coil 24. Typically the generated $\omega_1$ and $\omega_3$ currents are substantially equal in amplitude to and 180° out of phase with the mutually induced currents $I_{12}$ and $I_{32}$. It will be understood that coils 22 and 26 are driven by similar adaptive driver circuitry.

In other preferred embodiments of the present invention, signal processing circuitry 34 and computer 36 function to determine amplitudes and phases of the parasitic signal components generated by sensor coils 27, 28 and 29 due to mutual inductance among the radiator coils, and correct the determination of the position of probe 20 to account for the parasitic components.

In one such preferred embodiment of the present invention, the amplitude and phase of the magnetic field due to each of the coils is found using a harmonic detection method. In accordance with this method, the signals are sampled so that an integer number of periods of each of the signals are measured, i.e., $2\pi f_s/\omega = N$, where $f_s$ is the sampling frequency, and N is the number of sampling points. In this way, signal sampling is thus synchronized with the magnetic field frequencies. If N is a small number, then the sampling is preferably synchronized with the magnetic field, and may preferably be synchronized to take place at the peak of the periodic magnetic field. The signals thus obtained at a frequency $\omega$ may be expressed as:

$$U = \frac{2}{N} \sum_{n=0}^{N-1} A \sin\left(n\omega_{f_s} + \phi\right) \sin\left(2\pi n \frac{}{N}\right) = A\cos\phi$$

$$(7)$$

$$V = \frac{2}{N} \sum_{n=0}^{N-1} A \sin\left(n\omega_{f_s} + \phi\right) \cos\left(2\pi n \frac{}{N}\right) = A\sin\phi$$

In the presence of mutual inductance, the signals generated by the $j$th sensor coil comprise frequency components whose respective amplitudes Uij and Vij, at radiator coil frequency $\omega_i$, may be expressed as follows:

$$U_{ij} = A_{ij} \cos\phi_i + \sum_{k \neq i}^{3} A_{ijk}^{m} \cos\phi_{ik}^{m} \tag{8}$$

$$V_{ij} = A_{ij} \sin\phi_i + \sum_{k \neq i}^{3} A_{ijk}^{m} \sin\phi_{ik}^{m} \tag{9}$$

In equations (8) and (9), Aij and $\phi_i$ represent the amplitude and phase, respectively, of the position signal components received from sensor coil 28 (here identified as the $j$th sensor coil) at frequency $\omega_i$ due to the magnetic field of the $i$th radiator coil, disregarding mutual inductance effects. Aijk$^m$ and $\phi$ik$^m$ are the respective amplitudes and phases of the parasitic signal components generated by the $j$th sensor coil at frequency $\omega_i$, due to mutual inductance between the $i$th and $k$th radiator coils.

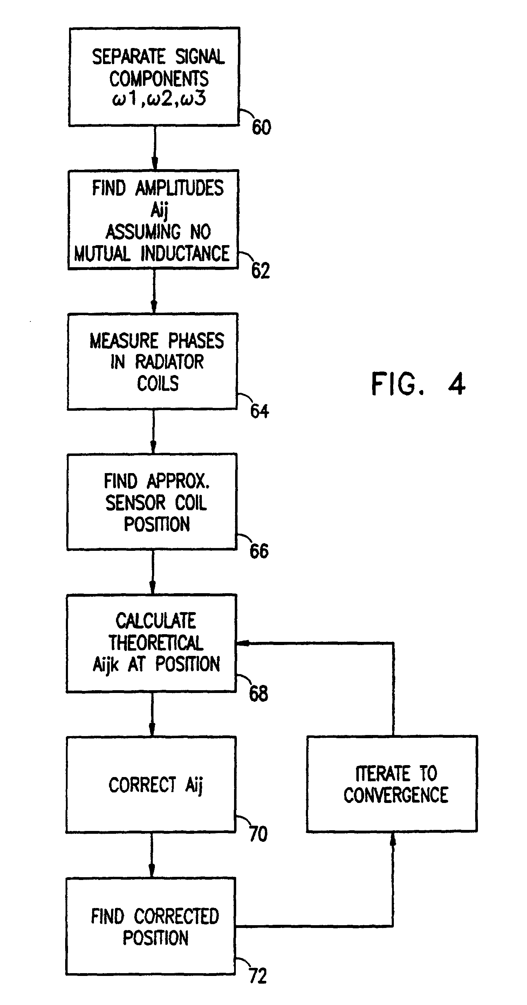

In order to correctly determine the position of sensor coils 27, 28 and 29 and probe 20, the signals received from the coil are preferably processed using the method shown in Fig. 4. First (60) the signals are separated into components Uij and Vij at frequencies $\omega_1$, $\omega_2$ and $\omega_3$ using methods known in the art, such as harmonic detection. The amplitudes of these signals Aij are then determined (62), assuming that mutual inductance effects are insignificant, i. e., that all Aijk$^m$ are zero. Phases $\varphi_i$ and $\varphi_{ik}{}^m$, which are dependent only on the radiator coils, are derived by measuring the respective radiator coil phases (64). The amplitudes Aij are then used to estimate the position and orientation of coils 27, 28 and 29 (66), as described, for example, in PCT patent application number WO 96/05768.

The theoretical values of Aijk$^m$ are then calculated for the estimated position of the sensor coils (68). To perform this calculation, the parasitic magnetic fields generated due to mutual inductance among the radiator coils, $H_{ik}$, are determined at the sensor coils' estimated position. These parasitic fields are transformed from the coordinate frame of the radiator coils to that of the sensor coils, based on the projections of the parasitic fields along the respective axes of the sensor coils. The field projections are used to calculate theoretical amplitudes of parasitic signal components generated by the sensor coils, using the relationship

$$A_{ijk}^{m} = \sum_{p=1}^{3} M_{jp} \, \overline{A}_{ipk}^{m} \tag{10}$$

where the three values of $\overline{A}_{ipk}^{m}$ are the theoretical signal amplitudes in the radiator coils' frame of reference, and Mjp are the elements of the orientation matrix.

Various methods may be used to determine the amplitude and direction of the parasitic magnetic fields $H_{ik}$ at the position of the sensor coils. In one preferred embodiment of the present invention, mutual inductances among the coils and the resultant magnetic fields are calculated theoretically from geometrical considerations, using methods well known in the art. In an alternative preferred embodiment, the mutual inductance of a pair of radiator coils is determined by activating the driver circuitry of one of the coils, so as to generate a magnetic field at its driving frequency, and then measuring electrical current flowing in the other coil at this frequency. These measured mutual inductances are then used to calculate the resultant parasitic magnetic fields, as explained above.

Finally, the theoretical Aijk$^m$ coefficients are substituted back into equations (8) and (9) to determine corrected values of the nine Aij elements (70), and these values are used to correct the position and orientation coordinates of the probe (72). Steps (68), (70) and (72) are preferably repeated iteratively until the position and orientation coordinates of the probe converge.

It will be understood that while the above method has been described with reference to a system comprising three radiator coils and three sensor coils, it may equally be applied to other electromagnetic object tracking system, using greater or fewer numbers of coils or antennae,

**Claims**

1. Apparatus for tracking an object by generating magnetic fields, comprising:

   a plurality of radiator coils (22, 24, 26);
   driver circuitry (30, 32, 33) coupled thereto, which drives the coils (22, 24, 26) so as to generate magnetic fields at a plurality of driving frequencies;

   wherein each of the plurality of radiator coils (22, 24, 26) generates a field substantially only at a single, respective driving frequency; **characterised by**:

   circuitry (32) associated with at least one of said plurality of radiator coils (24), wherein said circuitry (32) generates magnetic fields for substantially eliminating magnetic fields generated by the at least one coil (24) in response to fields generated by the other coils (22, 26).

2. Apparatus in accordance with claim 1, wherein said circuitry for eliminating comprises one or more shimming coils (40).

3. Apparatus in accordance with claim 2, wherein the one or more shimming coils (40) are interwound with the at least one of said plurality of radiator coils (24).

4. Apparatus in accordance with claim 2 or 3, wherein the driver circuitry (32) further drives one of the one or more shimming coils (40) associated with the at least one of said plurality of radiator coils (24) at the respective frequency of another one of said plurality of radiator coils (22).

5. Apparatus in accordance with any preceding claim, wherein the circuitry (32) for eliminating includes an adaptive current supply (52).

6. Apparatus in accordance with claim 5, wherein the adaptive current supply (52) generates electrical current in the at least one of said plurality of radiator coils (24) at the respective driving frequency of another one of said plurality of radiator coils (22).

7. Apparatus in accordance with claim 5 or 6, wherein the circuitry (32) for eliminating further includes a current analyzer (54) coupled to the adaptive current supply (52).

8. Apparatus in accordance with claim 7, wherein the current analyzer (54) measures parasitic electrical current flowing in the at least one of said plurality of radiator coils (24) at the respective driving frequency of another one of said plurality of radiator coils (22), and causes the adaptive current supply (52) to adjust the generated current so as to minimize the parasitic electrical current.

9. Apparatus in accordance with any preceding claim, wherein the driver circuitry maintains a constant current in each of the plurality of radiator coils (22, 24, 26) at the single, respective driving frequency thereof.

**Patentansprüche**

1. Vorrichtung zur Spurverfolgung eines Objekts durch Erzeugung von magnetischen Feldern, umfassend:

   eine Vielzahl von Strahlungsspulen (22, 24, 26);
   eine damit verbundene Treiberschaltung (30, 32, 33), die die Spulen (22, 24, 26) ansteuert, um magnetische Felder bei einer Vielzahl von Steuerfrequenzen zu erzeugen;

   wobei jede der Vielzahl von Strahlungsspulen (22, 24, 26) ein Feld bei im Wesentlichen einer einzelnen, jeweiligen Steuerfrequenz erzeugt; **dadurch gekennzeichnet, daß**
   die Schaltung (32) mit zumindest einer der Vielzahl von Strahlungsspulen (24) verknüpft ist, wobei die Schaltung (32) magnetische Felder zur im Wesentlichen Eliminierung von magnetischen Feldern, die durch zumindest eine Spule (24) in Reaktion auf Felder, die durch die anderen Spulen (22, 26) erzeugt werden, erzeugt.

**2.** Vorrichtung nach Anspruch 1, **dadurch gekennzeichnet, daß**
die Schaltung zur Eliminierung eine oder mehrere Trimmspulen (40) umfaßt.

**3.** Vorrichtung nach Anspruch 2, **dadurch gekennzeichnet, daß**
eine oder mehrere Trimmspulen (40) mit zumindest einer der Vielzahl von Strahlungsspulen (24) verzwimt ist bzw. sind.

**4.** Vorrichtung nach Anspruch 2 oder 3, **dadurch gekennzeichnet, daß**
die Treiberschaltung (32) ferner eine der einen oder mehreren Trimmspulen (40), die mit zumindest einer der Vielzahl von Strahlungsspulen (24) bei der jeweiligen Frequenz von einer anderen der Vielzahl von Strahlungsspulen (22) verknüpft ist, ansteuert.

**5.** Vorrichtung nach einem der vorangehenden Ansprüche, **dadurch gekennzeichnet, daß**
die Schaltung (32) zur Eliminierung eine anpassungsfähige Stromversorgung (52) enthält.

**6.** Vorrichtung nach Anspruch 5, **dadurch gekennzeichnet, daß**
die anpassungsfähige Stromversorgung (52) elektrische Ströme in zumindest einer der Vielzahl von Strahlungsspulen (24) bei der jeweiligen Steuerfrequenz einer anderen der Vielzahl von Strahlungsspulen (22) erzeugt.

**7.** Vorrichtung nach Anspruch 5 oder 6, **dadurch gekennzeichnet, daß**
die Schaltung (32) zur Eliminierung ferner einen Stromanalysator (54) enthält, der mit der anpassungsfähigen Stromversorgung (52) verbunden ist.

**8.** Vorrichtung nach Anspruch 7, **dadurch gekennzeichnet, daß**
der Stromanalysator (54) parasitären elektrischen Strom mißt, der zumindest in einer der Vielzahl von Strahlungsspulen (24) bei der jeweiligen Steuerfrequenz einer anderen der Vielzahl von Strahlungsspulen (22) fließt, und bewirkt, daß die anpassungsfähige Stromversorgung (52) den erzeugten Strom anpaßt, um den parasitären elektrischen Strom zu minimieren.

**9.** Vorrichtung nach einem der vorangehenden Ansprüche, **dadurch gekennzeichnet, daß**
Steuerschaltung einen konstanten Strom in jeder der Vielzahl von Strahlungsspulen (22, 24, 26) bei der einzelnen, jeweiligen Steuerfrequenz derselben beibehält.

**Revendications**

**1.** Dispositif pour suivre un objet en générant des champs magnétiques, comportant :

une pluralité de bobines rayonnantes (22, 24, 26),
un circuit d'entraînement (30, 32, 33) couplé à celles-ci, qui entraîne les bobines (22, 24, 26) de manière à générer des champs magnétiques à une pluralité de fréquences d'entraînement,
chaque bobine de la pluralité de bobines rayonnantes (22, 24, 26) générant un champ essentiellement uniquement à une fréquence d'entraînement respective unique, **caractérisé par** :

un circuit (32) associé à au moins une bobine de ladite pluralité de bobines rayonnantes (24), dans lequel ledit circuit (32) génère des champs magnétiques pour éliminer essentiellement des champs magnétiques générés par la au moins une bobine (24) en réponse à des champs générés par les autres bobines (22, 26).

**2.** Dispositif selon la revendication 1, dans lequel ledit circuit d'élimination comporte une ou plusieurs bobines de compensation (40).

**3.** Dispositif selon la revendication 2, dans lequel les unes ou plusieurs bobines de compensation (40) sont enroulées mutuellement avec la au moins une bobine de ladite pluralité de bobines rayonnantes (24).

**4.** Dispositif selon la revendication 2 ou 3, dans lequel le circuit d'entraînement (32) entraîne de plus une bobine parmi les unes ou plusieurs bobines de compensation (40) associée à la au moins une bobine de ladite pluralité de bobines rayonnantes (24) à la fréquence respective d'une autre bobine de ladite pluralité de bobines rayonnantes (22).

**5.** Dispositif selon l'une quelconque des revendications précédentes, dans lequel le circuit (32) d'élimination comporte une alimentation en courant adaptative (52).

**6.** Dispositif selon la revendication 5, dans lequel l'alimentation en courant adaptative (52) génère un courant électrique dans la au moins une bobine de ladite pluralité de bobines rayonnantes (24) à la fréquence d'entraînement respective d'une autre bobine de ladite pluralité de bobines rayonnantes (22).

**7.** Dispositif selon la revendication 5 ou 6, dans lequel le circuit (32) d'élimination comporte de plus un analyseur de courant (54) couplé à l'alimentation en courant adaptative (52).

**8.** Dispositif selon la revendication 7, dans lequel l'analyseur de courant (54) mesure un courant électrique parasite s'écoulant dans la au moins une bobine de ladite pluralité de bobines rayonnantes (24) à la fréquence d'entraînement respective d'une autre bobine de ladite pluralité de bobines rayonnantes (22), et amène l'alimentation en courant adaptative (52) à ajuster le courant généré de manière à minimiser le courant électrique parasite.

**9.** Dispositif selon l'une quelconque des revendications précédentes, dans lequel le circuit d'entraînement maintient un courant constant dans chaque bobine de la pluralité de bobines rayonnantes (22, 24, 26) à la fréquence d'entraînement respective unique de celle-ci.

FIG. 1

EP 0 892 908 B1

FIG. 2

EP 0 892 908 B1

FIG. 3

TO COIL 24

CURRENT ANALYZER — 54

ADAPTIVE CURRENT SUPPLY — 52

$\omega_1, \omega_3$

SYSTEM CURRENT SUPPLY — 50

$\omega_2$

32

FIG. 4